# EUROPEAN PATENT APPLICATION

(11) **EP 4 011 857 A1**
(43) Date of publication of application: **15.06.2022**
(21) Application number: 21212795.5
(22) Date of filing: 07.12.2021
(51) Int. Cl.: C07C 29/151, C07C 31/04, C07C 68/04, C07C 69/96

(54) **APPARATUS AND PROCESS FOR PRODUCING DIMETHYL CARBONATE**

(30) Priority: 09.12.2020 ES 202031224
(71) Applicant: Blueplasma Power, S.L., 12006 Castellón (ES)
(72) Inventor: ARAYA BRENES, MARIO, 12006 Castellón de la Plana (ES)
(74) Representative: Soler Lerma, Santiago

(57) **Abstract**

The present application relates to an apparatus and process for producing dimethyl carbonate, in particular a system (apparatus or process) for DMC synthesis without the need of using a dehydrating agent. More particularly, the feed mixture for the process can be selected from the following options: a) carbon monoxide, methanol and flue gas from the process, b) synthesis gas without CO₂ and flue gas from the process, c) synthesis gas with CO₂ and added synthesis gas from purified flue gas from the process. The process uses a catalyst cluster comprising a specific combination of different groups of heterogeneous catalysts wherein each group has a different function. Also the invention relates to an apparatus comprising a specific combination of heterogeneous catalysts for applying different routes to produce dimethyl carbonate from each feed mixture option, on continuous basis.

## Description

### FIELD OF THE INVENTION

The present invention relates to an apparatus and process for producing dimethyl carbonate, in particular a system (apparatus or process) for DMC synthesis without the need of using a dehydrating agent. More particularly, the feed mixture for the process can be selected from the following options: a) carbon monoxide, methanol and flue gas from the process, b) synthesis gas without CO₂ and flue gas from the process, c) synthesis gas with CO₂ and added synthesis gas from purified flue gas from the process. The process uses a catalyst cluster comprising a specific combination of different groups of heterogeneous catalysts wherein each group has a different function. Also the invention relates to an apparatus comprising a specific combination of heterogeneous catalysts for applying different routes to produce dimethyl carbonate from each feed mixture option, on continuous basis.

### BACKGROUND OF THE INVENTION

Dimethyl carbonate (DMC) is an organic compound with the formula OC(OCH₃)₂, it is a colorless, flammable liquid. It is classified as a carbonate ester. This compound has found use as a methylating agent and more recently as aprotic polar solvent which is capable to replace methyl ethyl ketone (MEK) and para-chloro benzotrifluoride, as well as tert-butyl acetate. DMC also has the characteristics to be used as an alternative fuel additive which is exempt from the restrictions placed on most volatile organic compounds (VOCs). The high amount of oxygen in DMC makes it as a suitable replacement of MTBE, a gasoline oxygenate. In the USA, dimethyl carbonate is often considered to be a green, environmentally neutral reagent. DMC as an intermediate in polycarbonate synthesis has found large captive use in the production of diphenyl carbonate through transesterification with phenol which is a widely used raw material for the synthesis of bisphenol-A-polycarbonate in a melt polycondensation process. Also, DMC has been employed as electrolytes in lithium ion batteries.

DMC has been produced by reacting methanol (MeOH), traditionally with phosgene and more recently with carbon monoxide (CO) by oxidative carbonylation. The major drawbacks of the former process are the high toxicity of phosgene and the disposal of the coproduced hydrogen chloride, while that of the latter is catalyst deactivation at high conversion besides the use of toxic CO and the high cost of oxygen.

The most important common methods to produce DMC from methanol and carbon oxides are the followings:
(1) Phosgenation of methanol:

   2CH₃OH + COCl₂ → (CH₃)₂CO + 2HCl

   Phosgenation of methanol was the most popular method of DMC synthesis before the 1980s, but due to toxicity of chlorine compounds, since the 1980s researchers mainly within the chemical industry have intensively developed non-phosgene routes to DMC synthesis.
(2) Oxidative carbonylation of methanol which is catalyzed by copper catalysts developed by ENIChem (Italy) and licensed to GE for the DPC (diphenyl carbonate) manufacturing process. This route follows the following chemical equation: 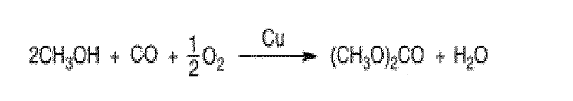 The reaction occurs in the liquid phase. A vapor-phase reaction process was also developed using solid catalysts such as CuCl₂ supported on activated carbon. MeOH, CO, and O₂ need to be supplied from outsources. The storage of CO and O₂ is in tanks under pressure.
(3) Carbonylation of methyl nitrate and decarbonylation of dimethyl oxalate (DMO). The methyl nitrate process developed by Ube Industry, Japan, is a variation of the direct methanol oxidative carbonylation process. Methanol is first oxidized to methyl nitrate with NO and O₂ under mild conditions (2-3 atm, 40 °C) in the absence of a catalyst. The methyl nitrate is separated from the coproduced water and carbonized by palladium (Pd²⁺) catalysts in the presence of O₂ to form DMC. The reaction occurs in the gas phase over a heterogeneous catalyst bed. Using palladium metal (Pd⁰) catalyst, methyl nitrate is carbonized to dimethyl oxalate (DMO), which can also be converted to DMC: 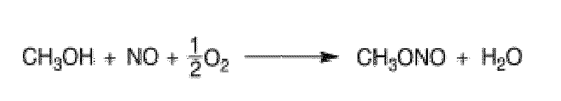 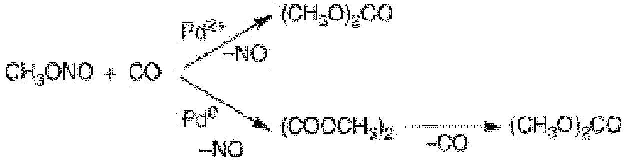 MeOH, CO, and O₂ need to be supplied from outsources. The storage of CO and O₂ is in tanks under pressure.
(4) DMC can also be produced industrially by a trans-esterification of ethylene carbonate or propylene carbonate and methanol, which also affords respectively ethylene glycol or propylene glycol.
(5) It is also possible to produce DMC by the methanolysis of urea. The tin-catalyzed reaction of methanol with urea to give DMC is a well-known synthesis method. The reactions of the process can be illustrated as follows:

   (NH₂)₂ CO + CH₃OH → H₂NCOOCH₃+ NH₃

   H₂NCOOCH₃ + CH₃OH →CH₃OCOOCH₃ + NH₃

   The overall reaction can be presented as follows:

   (NH₂)₂CO + 2CH₃OH → CH₃OCOOCH₃ + 2NH₃

An alternative route to produce DMC is a direct synthesis of DMC from methanol and CO₂. Homogeneous and heterogeneous catalysts are effective in this direct synthesis reaction. There are several reports where the reaction is carried out in a fixed bed reactor comprising various types of heterogeneous catalysts. Among them, metal oxides, in particular CeO₂ and ZrO₂ catalysts, were found consistently active in this reaction. Some others have demonstrated to produce DMC in high yields, among them carbon supported Cu/Cu-Ni, Cu-Ni or H₃PO₄ modified V₂O₅, metal oxide supported Rh, and heteropolyacids. The MeOH conversion reported in these works was in the range of 5-10% with mild-to-high DMC selectivity (60-90%), although a much lower methanol conversion value should be expected according to thermodynamics. Because of the thermodynamic constraints, the reaction between MeOH and CO₂ results in low yields of DMC in the common processes.

Moreover, CO₂ is kinetically inert and thermodynamically stable. Hence, CO₂ activation is the key problem in its conversion to DMC. The reaction between CH₃OH and CO₂ is slightly exothermic, with the heat of reaction of 23 kJ/mol. Hence to reach a higher amount of DMC yield, a lower reaction temperature is preferable. The low yield of DMC is due to the generation of H₂O as a by-product. However, higher pressure and instant removal of H₂O produced by employing suitable dehydrating agents can shift the equilibrium towards products side. Currently, the most innovative catalysts are: CeO₂, ZrO₂, CeO₂-ZrO₂, K₂CO₃, Cu-Ni/SBA-15, Nb₂O₅/CeO₂, Cu-Ni/graphite, metal tetra alkoxides, H₃PW₁₂O₄₀/ZrO₂. These are some of the major common catalyst compositions for the DMC synthesis. In all of these catalyst systems, H₂O as byproduct to DMC is an important factor reducing the overall yield. Therefore, all these processes are designed to avoid byproduct H₂O by using specific dehydrating agents.

Due to the fact that H₂O byproduct in the DMC production is the main enemy of the process efficiency, the most important existing heterogeneous catalyst synthesis to produce DMC require dehydrating agents such as homogenous dehydrating agents traveling throughout all the process. Then, after producing the DMC, the dehydrating agent requires to be changed (de-hydrated) or restored with special processes. This increases the costs of the common processes.

The following patents and research works have been published during the last decade, presenting ways to produce DMC using mainly CO₂ and methanol. Most of them with dehydrating agents and one of these patents comprises a circulating tube to extract out at least part of the water.

Patent No. US 9,249,082 B2 presented by Khalid A. Almusaiteer on February 2, 2016 describes a process for the synthesis of dimethyl carbonate from carbon dioxide and methanol. In this patent, the author removes at least a portion of the water by circulating the reaction mixture through a dehydrating tube.

Darbha Srinivas and Unnikrishnan Pulikkeel disclosed in patent No. US 9,073,849 B2 that DMC could be produced using calcined catalyst consisting of zirconium pyrophosphate derived from zirconium phosphonate and employing as water trapping agent one selected from the group of molecular sieves and 2,2-dimethoxy propane.

Ramaraos article on 2 March 2019 from the Catalysis and Fine Chemicals Department, CSIR-Indian Institute of Chemical Technology, Hyderabad, Telangana 500 007, India, describes a method of producing DMC from methanol and CO₂. They used ZnO-CeO2 catalysts prepared by a co-precipitation method and 2-cyanopyridine as dehydrating agent flowing together with the raw materials. The mentioned dehydrating agent 2-cyanopyridine is restored after dewatering the reaction to produce DMC.

Even though several non-reductive CO₂ transformation pathways have been presented to produce DMC from methanol and CO₂, high costs for these processes and additional process steps for restoring the de-hydrating agents are still needed. Thus, there is a demand for a novel technology and improved DMC synthesis processes.

### SUMMARY OF THE INVENTION

The above objective problem has been solved by the apparatus and the process as defined in the independent claims. Further embodiments and alternatives are described in the dependent claims and the following description.

More particularly, the apparatus for producing dimethyl carbonate (DMC) according to the present invention comprises a DMC reactor for synthesizing DMC from a feed mixture at least comprising MeOH, CO₂, and CO. The apparatus is further characterized in that the DMC reactor comprises a mixture of at least two heterogeneous catalyst groups comprising catalysts C2 and C3, wherein catalyst C2 promotes the reaction of MeOH with CO₂ to produce DMC and H₂O as byproduct, and catalyst C3 promotes the transformation of CO and H₂O to H₂ and CO₂.

The process according to the invention comprises the step of synthesizing DMC from a feed mixture at least consisting of MeOH, CO₂, and CO by heterogeneous catalysts in a DMC reactor comprising a mixture of at least two heterogeneous catalyst groups comprising catalysts C2 and C3. The catalyst C2 promotes a reaction of MeOH with CO₂ to produce DMC and H₂O as byproduct. The catalyst C3 promotes the transformation of CO and H₂O to H₂ and CO₂.

The apparatus and the process of the invention makes possible the production of DMC with competitive yields and selectivity without the need of dehydrating agent because of the specific combination of the at least two heterogeneous catalysts C2 and C3. Also, due to the heterogeneous catalyst system with at least the above-identified two different functionalities, there is no need of a separate dehydrating reaction in this process step and the DMC product is produced substantially water-free within the DMC reactor. Substantially water-free means in this regard that the DMC obtained at the end of the DMC reactor by condensing the DMC and separating the liquid DMC in a DMC tank is more than 90 %, preferably more than 95 %. The water content usually is lower than 5 %, sometimes only traces of water can be found in the end product without further separation steps. Additionally, further purification processes such as extractive distillation for separating DMC from MeOH and other liquid byproducts can be used to improve the purity if needed.

In addition, the apparatus as well as the process of the invention provides the chance of continuously synthesizing DMC from a mixture comprising at least MeOH, CO₂, and CO without the additional step of restoring any dewatering agents as needed in any of the prior art processes. The dewatering reaction occurs in the DMC reactor by the catalytic reaction promoted by the heterogeneous catalyst C3. This reaction is the transformation of CO and H₂O to H₂ and CO₂ occurring subsequent of the production of water or at the same time of generating water as by-product from the carbonylation reaction of MeOH in the DMC reactor. This immediate and effective dewatering action during the DMC formation, without using dehydrating agent flowing with raw material composed of MeOH, CO and CO₂ is based on the use of the two mentioned heterogeneous catalyst groups in the DMC reactor. Hence, the surprising effect achieved with the specific combination of above mentioned two catalyst groups is important for reducing the costs of the DMC production.

Moreover, utilization of carbon dioxide has gained considerable attention in many industrially relevant chemical reactions. This is largely driven by the fact that CO₂ is one of the most suspected greenhouse gases responsible for climate change and also by its increasingly vast availability from the CO₂ capture facilities. One promising strategy to convert a large amount of CO₂ is to produce fundamental and highly demanded chemicals like fuels such as methanol and DMC. Therefore, the apparatus and the process of the present invention can be used to lower the CO₂ content in the atmosphere or produced in chemical processes. In the present invention, CO₂ plays an important role as reactant in the synthesis of DMC by heterogeneous catalysis.

### PREFERRED EMBODIMENTS OF THE APPARATUS AND PROCESS

In the following, several preferred embodiments and alternatives are described which all can be used alone or in any combination. The description of the following embodiments shall be seen as a description of possibilities to supplement the apparatus or the process of the above-defined general inventive concept in the applications of the present invention. The apparatus as defined in its most general concept of the invention above usually is used in a complex reactor system with additional means for regulating the whole process. Some of these optional means are described as preferred embodiments in the following.

In such an embodiment of the apparatus according to the invention a MeOH reactor is comprised in this system. The methanol reactor is suitably placed upstream of the DMC reactor and is used for synthesizing MeOH from a feed mixture at least consisting of CO, CO₂, and H₂. Preferably, the feed mixture comprises as one component syngas, defined in this application as one which contains H₂ and CO in any proportion. The other component at least comprises CO₂ which is then used as reactant in the DMC synthesis in the DMC reactor. Further gas components such as flue gas from the output from the DMC reactor in raw or purified form can be present in the feed mixture of the MeOH reactor, if desired. This recycling of the flue gases can be used to increase the overall yield of the process of the present invention.

Alternatively, the MeOH reactor can integrally be placed within the DMC reactor. In this case, the DMC reactor preferably includes separated compartments for the respective reactions. Preferably the MeOH synthesis compartment is upstream to the DMC synthesis compartment as MeOH is used as one of the reactants in the DMC synthesis.

The MeOH reactor comprises a third group of heterogeneous catalysts, namely catalyst C1, which promotes the reaction of CO, H₂, and CO₂ to MeOH. The residual CO₂ is then used for the synthesis of DMC. If the MeOH reactor is integrally provided in the DMC reactor, the catalysts C1, C2 and C3 are placed mixed in the DMC reactor. It is preferred to arrange them in a specific combination as will be explained below in more detail.

Alternatively, the catalyst C1 is placed in a first zone A and catalysts C2 and C3 in mixed form are placed in a second zone B in either the DMC reactor integrally including the MeOH reactor or in two separate reactors, namely the MeOH and DMC reactors.

In a third alternative embodiment, catalyst C1 is placed in a first zone A and catalyst C2, optionally in mixed form with catalyst C3, is placed in a second zone B and catalyst C3 is placed in a third zone C in either the DMC reactor integrally including the MeOH reactor, or in two or three reactors, namely one MeOH reactor and two DMC reactors. In any of the last two alternatives, the catalyst C1 is placed upstream or at least inside the same reactor because this catalyst promotes the synthesis of MeOH which is one of the reactants of the following DMC synthesis processes.

As has been shown above, the present invention comprises an organized cluster of synergistically acting heterogeneous catalysts (HECATS). In mentioned alternatives, the HECATS are strategically combined and placed inside a reactor or reactor system consisting of at least one fixed-bed reactor in a continuous process without consuming dehydrating agent, for producing water-free DMC from a feed mixture.

At least the DMC reactor can suitably be configured as a reactor working at low pressure (approx. 60 bars) and low temperature (below 200 °C). The DMC reactor has at least one heat exchanger system at the output. This is capable to be used to lower the temperature of the product stream, in order to condense the liquid portion and separate it from the output gas components.

The heat exchanger at the output of the DMC reactor is suitably followed by at least one DMC condenser and DMC tank for cooling the produced DMC and separating it from the gaseous feed products and gaseous byproducts. The DMC is sufficiently water-free when condensed and can be stored in the DMC tank. Further purification steps can be applied if needed.

After condensing the DMC, the gaseous feed products and gaseous byproducts can be further fed into a MeOH condenser for cooling the byproduct MeOH and separating it from the gaseous feed products and byproducts. The condensed MeOH preferably is stored in a MeOH tank downstream of the DMC condenser. The apparatus optionally comprises a pipe connection between the MeOH tank and a feed pipe of the DMC reactor. This pipe connection can be used to recycle the MeOH byproduct stored in the MeOH tank in liquid form, in the DMC reactor, thus enhancing the yield of DMC synthesis in the DMC reactor.

In addition to the MeOH recycling connection, a further recycling connection can be used to increase the yield of the DMC synthesis. For this purpose, the MeOH condenser is connected to a feed pipe of the MeOH reactor by a pipe connection such that the untreated gaseous feed or gaseous byproducts are fed as recycled feed mixture. This recycled feed mixture is also called raw-flue gas (R-FLG). Optionally the raw-flue gas is admixed with fresh feed gas consisting of a mixture mainly comprised of CO and/or CO₂, and H₂. Syngas generated from organic waste with or without admixture of CO₂ preferably is used as the feed gas from environmental aspects.

An additional bypass line including a reforming unit to convert CO₂ and hydrocarbons to more syngas (CO+H2) and a pipe connection between the MeOH condenser or tank and the feed pipe of the MeOH reactor can be used to recycle raw-flue gas produced in the DMC reactor and transforming it into purified flue gas (P-FLG). The P-FLG can again be used as feed gas for the MeOH reactor, similar to the external syngas. This allows a great variety of feeding gas options as will be described later in this application.

According to an embodiment of the apparatus according to the invention, the MeOH reactor and/or the DMC reactor are shell-tube reactors having one or more tubes which are covered by a shell. Generally, the catalysts are placed inside of the one or more tubes and these tubes are optionally surrounded by a circulating heat transfer media to absorb excess heat generated by exothermic reactions in the reactors.

A preferred heterogeneous catalyst material for catalyst C1 is a mixture of CuO and ZnO, supported on Al₂O₃.

Exemplified materials for catalyst C2 are selected from the group consisting of CeO₂, ZrO₂, ZnO-CeO₂, and any mixture thereof.

Catalyst C3 is, for example, selected from the group consisting of Pt/γ-Fe₂O₃, Au/γ-Fe₂O₃, Au/CeO₂-MOₓ/Al₂O₃ (wherein M is at least one of the following group: La, Ni, Cu, Fe, Cr, and Y), Au-Y/CeO₂, Pt-Y/CeO₂, Au/CeO₂-ZrO₄, Au/Hydrotalcite with Cu-Zn-Al₂O₃, Pt/Hydrotalcite with Cu-Zn-Al₂O₃, and Pt/CeO₂.

The preffered content of the catalysts C1, C2, and C3 in the overall composition of the catalysts, in weight percent, are 20 to 35% for C1, 15 to 35% for C2 and 15 to 60% for C3.

The process for producing dimethyl carbonate (DMC) according the present invention comprises a step of synthesizing DMC from a feed mixture at least consisting of MeOH, CO₂, and CO by heterogeneous catalysis in a DMC reactor. The process is characterized by the use of a specific heterogeneous catalysts mixture in the reactor system defined above. The mixture of at least two heterogeneous catalyst groups comprises catalysts C2 and C3 as defined above.

More particularly, the catalyst C2 promotes a reaction of MeOH with CO₂ to produce DMC and H₂O as byproduct. More particularly, the function of C2 is to promote the production of DMC by the reaction of MeOH and carbon dioxide (CO₂) coming from the FEEG:

2(CH₃OH) + CO₂ → (OCH₃)₂CO + H₂O

In this reaction H₂O is produced as the main byproduct. Generally, the reaction is an equilibrium reaction and has low yields at high temperatures. The yield of DMC can be increased either by lowering the temperature in the reactor or by trapping H₂O. H₂O needs to be removed from the equilibrium because it deactivates catalysts and retards the reaction for producing DMC.

According to the process of the invention, the produced H₂O and CO coming from the feed gas mixture are both reactants in the additionally occurring reaction in the DMC reactor. This additional reaction is heterogeneously catalyzed by the catalyst C3. The catalyst C3 promotes the transformation of CO and H₂O to H₂ and CO₂. This reaction is also known as water gas shift reaction (WGSR). More particularly, the function of C3 is to promote WGSR, preferably the ultra-low temperature ULT-WGSR, to remove the H₂O molecules formed mainly by the above mentioned produced DMC and any other water molecules coming in the feed gas and others left from the produced MeOH. This ULT-WGSR occurs as described in the following chemical equation:

CO + H₂O → CO₂ + H₂.

In this invention, ULT-WGSR replaces any dehydrating agent flowing with the FEEG when producing DMC. Therefore, in the present invention, H₂O is being removed by the ULT-WGSR when C2 is promoting the production of DMC wherein H₂O is produced as a byproduct.

According to a preferred embodiment of the process of the invention, the process comprises a step of synthesizing MeOH from a feed mixture at least consisting of CO, CO₂, and H₂, in a MeOH reactor. The MeOH reactor is either integrally combined within the DMC reactor or is sequentially placed upstream of the DMC reactor to produce MeOH for the DMC synthesis process in the DMC reactor. The step of synthesizing MeOH is a heterogeneous catalytic process catalyzed by a third group of heterogeneous catalysts, namely catalyst C1. Thus, the function of C1 is to promote the production of MeOH from the FEEG by the reaction of carbon monoxide (CO) and hydrogen (H₂) coming from the FEEG in line with the following chemical reaction:

CO + 2H₂ → CH₃OH

At the same time, the same catalyst C1 promotes the reaction between CO₂ and H₂ also from the FEEG, by the following reaction:

CO₂ + 3H₂ → CH₃OH + H₂O

The produced water (H₂O) and the incoming CO from the FEEG are both reactants in the low temperature WGSR which is also promoted mainly by C1 or a mixture of C1 and C3 if lower temperature of the reaction is desired:

CO + H₂O → CO₂ + H₂

According to this invention, ULT-WGSR promoted by C3 replaces any dehydrating agent flowing with the FEEG when producing DMC. Therefore, in the present invention, H₂O is being removed by the ULT-WGSR when C2 is promoting the production of DMC wherein H₂O is produced as a byproduct.

Good results can be achieved in the ULT-WGSR promoted by C3 at a temperature from 80 to 160 °C and this is desired because higher temperatures tend to decompose the DMC molecules and also affect negatively the reaction to produce DMC. Therefore, in order to remove the water molecules formed in the above mentioned reactions and the ones coming as contaminant in the FEEG, ULT-WGSR should work at temperatures similar to the ones required to produce DMC.

Summarizing the above chemical reactions occurring in this embodiment of the process of the present invention including reactions with the three catalysts C1, C2, and C3, the following reaction schemes describe the main reactions within the MeOH and DMC reactors or the DMC reactor alone if all three catalysts are incorporated in one reactor.

Reactions from catalyst C1 are herein named as R1-a and R1-b:

R1-a : CO + 2H₂ → CH₃OH

R1-b: CO₂ + 3H₂ → CH₃OH + H₂O

These two reactions are both exothermic reactions generating heat in the reactor system.

The following reaction from catalyst C2 group is herein named as R2:

R2 : 2(CH₃OH) + CO₂ → (OCH₃)₂CO + H₂O

This reaction is exothermic as well.

The following reaction from catalyst C3 group is herein named as R3:

R3: CO + H₂O → CO₂ + H₂

This reaction is exothermic as well.

According to a further preferred embodiment, the MeOH reactor is fed with a mixture of H₂ in the range of 3 to 10 wt.%, CO in the range of 40 to 69 wt.% and CO₂ in the range of 18 to 42 wt.%. Preferred pressure conditions for catalyst C1 are from 15 to 60 bars and temperature conditions are from 175 to 270 °C in order to produce MeOH from the feed gases as described above. The subsequent DMC reactor is fed with the output of the MeOH reactor at pressure conditions from 15 to 60 bars and temperature conditions from 130 to 165 °C for catalyst C2 and from 110 to 175 °C for catalyst C3, if the catalysts C2 and C3 are placed in separate conditions.

In case the MeOH reactor is integrally combined within the DMC reactor, the reactor preferably is fed with a mixture of H₂, in the range of 3 to 10 wt.%, CO in the range of 40 to 69 wt.%, and CO₂ in the range of 15 to 42 wt.%. More preferred ranges are H₂ in the range of 5 to 10 wt.%, CO in the range of 40 to 69 wt.%, and CO₂ in the range of 18 to 42 wt.%. In this reactor, the catalysts C1, C2, and C3 are mixed together. Thereby the preferred pressure conditions are from 15 to 60 bars and the preferred temperature conditions are from 130 to 175 °C in order to achieve suitable de-watering functionalities in the reaction zones.

The process of the invention is further more preferably carried out with a weight hourly space velocity of the feed gas in a range of about 1,200 to 125,000 h⁻¹, resulting in a continuous process of synthesizing DMC with high productivity and substantially water-free.

As explained above, the process does not need any dehydrating agent flowing with the raw material. Thus, the apparatus and process herein presented are based on a different dewatering reaction to the common synthesis methods for producing DMC. They not only meet the above outlined general needs, but provide further advantages. For example, the process as described before allows producing DMC from syngas (CO and H₂) and CO₂, and also from MeOH, CO and CO₂ without using O₂ in the FEEG.

Another advantage is that the apparatus as described above and in the claims allows developing a flexible process for producing DMC from different FEEG compositions:
a) SG containing mainly CO, H₂ and CO₂ such as the one coming from gasification,
b) CO₂ and SG containing mainly CO and H₂ such as the one coming from hydrocarbon steam reforming, and
c) MeOH, CO₂ and CO without integrating O₂ in the FEEG.

Hence, the high flexibility in the reactants (feed components) makes the apparatus and the process of the invention advantageous over the common processes. Also, the apparatus is adjusted for DMC synthesis using possible routes for using recycled flue gas (FLG) as raw material. There are at least the following three groups of feeding gas sources applicable in the apparatus and process of the present invention:
Group 1, mainly comprising: i) SG defined in this document as one which contains mainly H2 with CO in any proportion; ii) CO₂; iii) Purified flue gas P-FLG from the synthesis of the DMC process and defined in this document as one which contains mainly CO, H₂, traces of nitrogen (N₂) and a small amount of methane (CH₄); and iiii) methanol (MeOH) byproduct.
Group 2, mainly comprising: i) SG as it was defined above in group 1; ii) Recyclable Flue Gas (R-FLG) defined in this document as one which contains mainly CO₂, CO, H₂, traces of N₂ and a small amount of CH₄; iii) MeOH byproduct: Initially, while no R-FLG is flowing from the DMC reactor, this FEEG requires to be supplied of CO₂ from an outsource, afterward, when the R-FLG starts flowing from the DMC reactor, this CO₂ supplied from an outsource is, at least partly, replaced by the CO₂ byproduct which comes as part of the R-FLG.
Group 3, mainly comprising: i) MeOH from outsource and MeOH byproduct; ii) CO from outsource; and iii) R-FLG as it is defined above in group 2. Initially, while no R-FLG is flowing from the DMC reactor, this FEEG requires to be supplied of CO₂ from outsource, afterward, when the R-FLG starts flowing from the DMC reactor, this CO₂ supplied from an outsource is, at least partly, replaced by the CO₂ byproduct which comes as part of the R-FLG.

In an especially preferred embodiment, the HECATS are combined in a special manner to provide the above identified advantageous effects. HECATS can be placed mixed or in sequence inside the one or more reactors. If the HECATS are placed in sequence, C1 is placed as first in the sequence to promote the production of MeOH, by means of reactions R1-a and R1-b; C2 mixed with C3 are placed as second catalysts in the sequence to promote the production of DMC by means of R2, wherein H₂O is a byproduct which reacts with CO in the WGSR by means of C3 to dewater the zone where DMC is being produced, thus permitting a reasonable DMC yield in R2.

The process can be carried out in one or more reactors arranged in sequence or in parallel or in combination of both.

The working conditions can have an impact insofar that the DMC yield depends of the FEEG composition, gas hourly space velocity of the FEEG, on how the three HECATS catalysts are placed inside the reactor, the amount of each type of catalyst participating in the reactions, and also of the temperature and pressure at which each of the three types of catalysts reacts. Further influences may apply.

The preferred working conditions are:
- FEEG weight range composition is: hydrogen (H₂) in the range of 3 to 10%, carbon monoxide (CO) in the range of 40 to 69% and carbon dioxide (CO₂) in the range of 15 to 42%.
- Gas hourly space velocity (GHSV) range is from 1,200 to 125,000 h⁻¹.
- HECATS are placed inside the one or more reactors in sequence or in parallel or in a combination of both arrangements. The at least one or more reactors are followed by at least one or more cooling system or unit.
- Weight ranges for each type of catalyst participating in the HECATS are: C1 from 25 to 35%, C2 from 20 to 30% and C3 from 30 to 60%.
- Temperature ranges are from-175 to 270 °C for C1, from 130 to 175 °C for C2 and from 110 to 175 °C for C3.
- Pressure range is from 15 to 60 bars.

### EXAMPLE

The gasification of 1 kilogram of average plastic waste (polyethylene) reacting with water steam produces 2.35 NM3 of syngas mainly containing H₂, CO and CO₂ within the ranges mentioned above, which is capable to produce approximately 1.16 kg of DMC and 0.115 kg of MeOH under the following conditions: 38 bars, 110 to 240 °C, and 4,200 h⁻¹ GHSV. FLG left from the DMC production is mainly composed of CO₂ and some gases that have not been converted to DMC: H₂, CO, N₂, CH₄ and some MeOH.

Variations to the above example can be made, for example, gases to replace the SG from the gasification can be bought or obtained from other processes such as steam reforming of hydrocarbons. Also, MeOH vapor accompanied with CO₂, and CO, are able to replace the SG from the gasification process. These variations have the advantage of not having N₂ and CH₄ in the flue gas (FLG). However, if using flue gas for feeding the DMC reactor the costs for the raw materials can be lowered.

In a further preferred embodiment, the separation procedure is especially adapted. DMC with some MeOH condenses first in at least one tank and then MeOH condenses in at least a second tank, from which it flows in liquid form to the DMC reactor by means of a pump. In order to obtain pure DMC, MeOH that forms an azeotrope with DMC is normally separated by extractive distillation.

In order to make the rest of the Flue Gas (FLG) a recyclable flue gas (R-FLG) for the DMC synthesis process of the invention, N₂ is separated by means of a commercial membrane or a pressure swing absorption unit (PSA).

### DRAWING

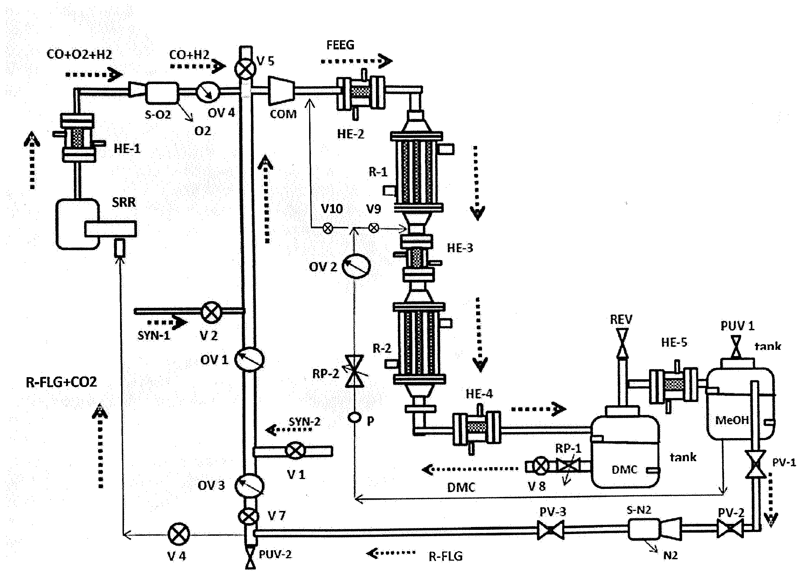

Abbreviations used in the drawing herein appears in yellow box.

In the following the apparatus and the process of the present invention will be explained by explaining an exemplary embodiment of the apparatus of the invention. The drawing and respective description of this exemplified embodiment are to be regarded as illustrative and not restrictive. An explanation of how each loop works is herein below after the general description of the drawing.

The Figure describes a DMC synthesis system including the apparatus for producing DMC according to the present invention. The apparatus comprises a compressor COM for feed gas FEEG , a heat exchanger HE-2, a MeOH reactor R-1 defining a zone A, a heat exchanger HE-3, a DMC reactor R-2 defining a zone B, a heat exchanger HE-4, a dimethyl carbonate (DMC) tank for liquid DMC with relieve valve REV, a heat exchanger HE-5, a MeOH tank for liquid MeOH with purge valve PUV 1. In zone A, the catalyst C1 is placed in the MeOH reactor R-1. In zone B, the catalysts C2 and C3 are placed within the DMC reactor R-2.

Additional connecting lines between the MeOH tank and the compressor COM for recycling the gaseous by-products to the compressor COM for feeding the gas into the MeOH reactor R-1 are provided. This connecting line comprises several pressure valves PV-2, PV-3, valves V1, V2, V7,, one way valves OV 1, OV 3, N2 separator S-N2, and feeding lines for syngas with CO₂ (SYN-1) and syngas without CO₂ (SYN-2). An additional bypass is provided by opening valve V4 after pressure valve PV-3 and closing V7. Thus, recycled purified flue gas P-FLG can be led through a bypass connecting line to compressor COM.

The bypass connecting line comprises a steam reforming reactor SRR (cracking unit) for converting the CO2 and CH4 coming from the recyclable flue gas R-FLG to syngas (CO+H2) with some contamination of O2, a heat exchanger HE-1, an O₂ separator SO2, and a one way valve OV4. The one way valve is connected to the inlet of the compressor COM for feeding the MeOH reactor R-1.

Another connecting line between the bottom outlet of the MeOH condenser or tank and the inlet to heat exchanger HE-3 for recycling liquid MeOH into the DMC reactor is provided with a pump P, a pressure reducer RP-2, and a one way valve OV 2. This line can be used to recycle the produced and separated MeOH for the DMC synthesis.

The liquid DMC product can be collected via an outlet for DMC with pressure reducer RP-1 and a valve V 8 attached to the DMC tank.

In the following, a description of the process is given together with the explanation of the apparatus while using three variations in feed compositions.

### PROCESS DESCRIPTION ON ROUTE/LOOP 1 (for using SG with CO₂)

1. In this loop, the SG containing CO₂ enters to the system through V2 and flows toward the compressor.
2. The flue gas (FLG) flows from the MeOH tank, passes through the N₂ separator S-N2 becoming recyclable flue gas (R-FLG), also through a commercial CO₂ plasma cracking torch steam reforming reactor (SRR) followed by a heat exchanger HE-1 for cooling purposes and a commercial O₂ separator S-O2 which works based in a membrane or a pressure swing adsorption unit. At this point the R-FLG becomes syngas herein named purified flue gas (P-FLG) and flows as part of the FEEG to the compressor.
3. Accordingly, the compressor is receiving two streams: one comprising the P-FLG and another one comprising the syngas SG with CO₂ (SYN-1). The sum of both streams composes the FEEG from which the DMC is produced.
4. After the FEEG is compressed in the compressor COM and preheated with heat exchanger HE-2, it passes through zone A in reactor R-1 which works at a temperature below 270°C and a pressure of about 60 bars for converting part of the FEEG in MeOH using C1 catalyst or C1 and C3 mixed together. Then the non-converted FEEG and the MeOH are cooled in the heat exchanger HE-3 to approximately 160 °C so as to pass to zone B in reactor R-2, in which the synthesis of DMC occurs by using C2 and C3 catalysts. The pressure conditions are also at about 60 bars. MeOH is a byproduct in this process.
5. Once the FEEG is converted to DMC and methanol as a byproduct, it passes through a heat exchanger HE-4 for cooling and condensing the DMC first. DMC after condensing falls into an end-product DMC tank from where the flue gas (FLG), containing the non-converted SG, the CO₂ byproduct and methanol, is separated from the DMC and flows through a heat exchanger HE-5 for cooling further the stream so as to separate the MeOH from the FLG and to condense it in the MeOH tank. Therefore MeOH in liquid form falls in this second tank from which it returns to HE-3 through V9 with V10 closed and it enters to the DMC reactor R2, by means of a pump P; the FLG becomes R-FLG by passing through the N₂ separation process by means of a commercial membrane or a pressure swing unit and then it flows to the compressor.
6. In this loop 1, valve V2 and V4 work in open position while V1 and V5 work in closed position (see drawing). Purge valves (PUV 1, PUV 2) changes from closed to open position only in case byproducts, such as CH₄ and/or N₂, are over a desired level.

### PROCESS DESCRIPTION ON ROUTE/LOOP 2 (for using SG without CO₂)

1. In this loop 2, the SG without CO₂ enters to the system through V2 and flows toward the compressor COM.
2. The FLG flows from the MeOH tank, passes through the N₂ separator S-N2 becoming R-FLG and passes through valve V7 to join the entering of the stream of SG without CO₂ (SYN-2).
3. Therefore, the compressor is receiving two streams: one comprising the R-FLG and another one comprising the SG without CO₂ coming from (SYN-2). The sum of both streams composes the FEEG from which the DMC is produced.
4. After the FEEG is compressed and preheated with heat exchanger HE-2, it passes through zone A in reactor R-1 which works at a temperature below 250 °C and a pressure of about 60 bars for converting part of the FEEG in MeOH using C1 or C1 mixed with C3 catalysts. Thereafter the non-converted FEEG and the MeOH are cooled in the heat exchanger HE-3 to approximately 160 °C so as to pass to zone B in reactor R-2 where the synthesis of DMC occurs by using C2 and C3 catalysts. The pressure conditions are also at about 60 bars. MeOH is a minor by product in this process.
5. Once the FEEG is converted to DMC and methanol as a byproduct, it passes through a heat exchanger HE-4 for cooling and condensing the DMC first. DMC after condensing falls into an end-product tank, DMC tank, from where the FLG is separated from the DMC and flows through a heat exchanger HE-5 for further cooling the stream so as to separate the MeOH from the rest of the FLG and condense it in the MeOH tank. Therefore MeOH in liquid form falls in this second tank from which it returns to HE-3 through V9 with V10 closed and it enters to the DMC reactor R-2 by means of a pump P. The FLG passes directly to the compressor COM.
6. In this loop 2, valves V2, V5 and V4 work in closed position while V1 works in open position. Purge valves PUV 1, PUV 2 change from closed to open position only in case byproducts, such as CH₄ and/or N₂, are over a desired level.

### PROCESS DESCRIPTION ON ROUTE/LOOP 3 (for using MeOH and CO, also CO₂ for starting the process only)

1. MeOH and CO, coming from an external source, they both enter to the compressor COM through valve V5.
2. The FLG after the N₂ separator S-N2 unit becomes recyclable flue gas R-FLG and it passes through V7 and also enters to the compressor COM.
3. Therefore, the compressor is receiving two streams: one comprising the R-FLG and another one comprising the MeOH with CO. The sum of both streams composes the FEEG from which the DMC is produced.
4. After the FEEG is compressed and preheated to about 160 °C with heat exchanger HE-2, it passes through zones A and B wherein zone A operates as an extension of zone B and heat exchanger HE-3 works not as a heat exchanger but as a mere communication between the zones A and B. This configuration works at a pressure of about 60 bar for converting, in first pass part of the FEEG in DMC by using C2 and C3 catalysts. MeOH and CO₂ are minor byproducts in this process.
5. Once the FEEG is converted to DMC containing MeOH and CO₂ as byproducts, it passes through a heat exchanger HE-4 for cooling and condensing the DMC first. DMC after condensing falls into an end-product tank, namely DMC tank, from where the FLG is separated from the DMC and flows through a heat exchanger for cooling further the stream so as to condense the MeOH and to separate it in a second tank for MeOH, from the rest of the FLG. Therefore MeOH in liquid form falls in this MeOH tank from which it returns to HE-2 through V10 with V9 closed and it enters to the MeOH reactor R-2 by means of a pump P. The FLG passes directly to the compressor COM.
6. In this loop 3, valves V1, V2 and V4 work in closed position while V7 and V5 work in open position. Purge valves PUV 1, PUV 2 change from closed to open position only in case byproducts, such as CH₄ and/or N₂, are over a desired level.

A more preferred embodiment for carrying out the DMC synthesis herein presented comprises the above-identified three routes that can work independently or in combination of any of these routes. The route to be preferably used depends of the type and amount of gas coming from outsource to feed the system.

In the first route (loop1), where the FEEG comprises SG with CO₂ and P-FLG, the SG with CO₂ comes from an out source through valve 4. The flue gas FLG containing small amount of N₂ which is separated by means of a commercial membrane or pressure swing unit, and becomes recyclable flue gas R-FLG. The small amount content of CH₄ is maintained at a low level by passing the R-FLG through a steam reforming reactor SRR (cracking unit) for converting the CO2 and CH4, coming from the recyclable flue gas R-FLG, to syngas (CO+H2) with some O2 as by product. And, after passing this cracking unit, the stream is cooled in a heat exchanger and then it flows through a commercial membrane or pressure swing unit to separate the produced O₂ left from the cracking process and then the R-FLG becomes P-FLG which is composed of syngas and joins the feeding gas stream containing SG with CO₂. MeOH byproduct is pumped from the MeOH tank to the DMC reactor, preferable after zone A and before zone B.

In loop 1, if after N₂ separation the amount level of N₂ is still more than desired, then purging/venting is necessary and part of the recyclable flue gas R-FLG passes through purge valve 2 PV2 (see drawing) and continues its Loop by passing through valve V4 while V7 is closed.

In the second route (loop 2) where the SG without CO₂ feeds the system, any contamination in the FLG such as N₂ and/or CH₄ is maintained at a low level by purging/venting the system through purge valve 1 PV1. Then recyclable flue gas R-FLG passes through valve V7 while V4 is closed (see drawing) and joins the SG without CO₂ and CH₄ before entering to the compressor COM and passes through the DMC reactor. MeOH byproduct is pumped from the MeOH tank to the HE-3 through V9 with V10 is closed and it enters to the DMC reactor.

In the third route (loop 3), where the FEEG components are the recyclable flue gas R-FLG, MeOH and CO, the R-FLG is free of N₂ and CH₄ and it is able to feed the system (apparatus) and produce DMC without requiring any separation in the FLG. For starting, in this loop, CO₂ from outsource is required to start the process. MeOH byproduct is pumped from the MeOH tank to the HE-2 through V10 with V9 closed and it enters to the MeOH reactor 1 R-1 by means of a pump P. The FLG passes directly to the compressor COM.

In loop 3, if there is any amount level of infiltrated N₂ in the system (apparatus) and this level of N₂ is more than desired, then purging part of the FLG is necessary by passing it through purge valve 1 V1 (see drawing) and then the FLG continues its loop 3 by passing through valve V7 while V4 is closed. Alternatively, if N₂ level is high, it can be lowered by activating the N₂ separator S-N₂

In a further embodiment of the apparatus and process of the invention, the preferred, but not the only type of reactor for producing DMC by using the three HECATS, is the shell-tube reactor where catalysts are placed inside of the one or more tubes covered by the shell, wherein these tubes are surrounded by a circulating heat transfer media to regulate the temperature of the reactor. One or more reactors can be placed in parallel or in sequence and it is preferred that each one has at least one heat exchanger system for the tubes hosting the HECATS which are surrounded by a circulating heat transfer media. Each reactor has at least one zone for hosting one or more of the three HECATS. When HECATS are placed in sequence, at least three heat exchangers are preferred: one in zone A, another one in zone B and a third one between zones to avoid exposing the reactions performance to a different temperature than the required one. It is preferred that each reactor has at least one heat exchanger for the tubes hosting the HECATS which are surrounded by a circulating heat transfer media and at least one at the output and at least one zone for hosting one or more of the three HECATS. When HECATS are placed in sequence, at least two heat exchangers are preferred for each zone: one for the tubes hosting the HECATS which are surrounded by a circulating heat transfer media and at least another one at the output. Also, optionally, at least one heat exchanger between zones is necessary .

The manner to place the HECATS inside the reactor are in a mixed way or in sequence. Each reactor has at least one zone to place one or more of the three HECATS catalyst. There is free communication among all zones and reactors. It is preferred that each zone has independent heat exchangers in order to operate at an optimum temperature and should have at least one second inlet for feeding it with MeOH.

Therefore, the at least one or more reactors to carry out the process to produce DMC, should maintain about same low pressure range of 15 to 60 bars in all zones and all reactors have the way to adjust a specific temperature for each of its zones. When HECATS are placed in sequence: a) Zone A is where C1 is placed and the reactor is designed to work in the preferred temperature range of 175 to 270 °C to mainly produce MeOH, b) Zone B is where C2 is placed mixed with C3 and the reactor is designed to work in the preferred temperature range of 110 to 175 °C to produce ultra-low WGSR for producing H₂O free DMC.

WGSR in most reactors normally occurs above 230 °C. Little work has been done in developing catalysts to promote WGSR at ultralow temperatures: below 160 °C. The reason is that industries requiring this reaction type are mainly the ones interesting in producing MeOH and H₂ and they don't need ultra-low WGSR temperatures in their processes. The preferred catalysts herein defined for C3 have shown to work well and fast for promoting ultra-low WGSR in the range of about 110 to 175 °C and this is the same temperature range that C2 needs to promote the R2 reaction. Industrially it has never been used to replace dehydrating agents for producing DMC. Nor the use of syngas in the production of DMC has been considered.

The above described invention can therefore be applied in organic synthesis, especially using to take CO₂ trapped in a waste product and re-use it to build useful chemicals. Recent advances in organometallic chemistry and catalysis provide effective means for the chemical transformation of CO₂ and its incorporation into synthetic organic molecules under mild conditions. CO₂ as a renewable one-carbon building block in organic synthesis could contribute to a more sustainable use of resources. The process of the present invention provides a suitable way to these building-blocks.

The present invention comprises the apparatus to carry out the DMC synthesis with a specific combination of HECATS to produce DMC, the procedure to place mentioned HECATS in the apparatus, the apparatus for its synthesis and the possible routes to use derived FLG as raw material in the apparatus and process of the present invention as described herein before.

## Claims

1. Apparatus for producing dimethyl carbonate (DMC), comprising a DMC reactor for synthesizing DMC from a feed mixture at least comprising MeOH, CO₂, and CO, wherein the DMC reactor comprises a mixture of at least two heterogeneous catalyst groups comprising catalysts C2 and C3, wherein catalyst C2 promotes the reaction of MeOH with CO₂ to produce DMC and H₂O as byproduct, and catalyst C3 promotes the transformation of CO and H₂O to H₂ and CO₂.

2. Apparatus according to claim 1, further comprising a MeOH reactor for synthesizing MeOH from a feed mixture at least consisting of CO, CO₂, and H₂, wherein the MeOH reactor is either integrally combined within the DMC reactor or is sequentially placed upstream of the DMC reactor, wherein the MeOH reactor comprises a third group of heterogeneous catalysts, namely catalyst C1, which promotes the reaction of CO, H₂ and CO₂ to MeOH.

3. Apparatus according to any of the preceding claims, wherein catalysts C1, C2 and C3 are placed in the DMC reactor integrally including the MeOH reactor, or catalyst C1 is placed in a first zone A and catalysts C2 and C3 in mixed form are placed in a second zone B in either the DMC reactor integrally including the MeOH reactor or in two separate reactors, namely the MeOH and DMC reactors, or catalyst C1 is placed in a first zone A and catalyst C2, optionally in mixed form with catalyst C3, is placed in a second zone B and catalyst C3 is placed in a third zone C in either the DMC reactor integrally including the MeOH reactor, or in two or three reactors, namely one MeOH reactor and two DMC reactors.

4. Apparatus according to any of the preceding claims, wherein at least the DMC reactor works at low pressure and low temperature and comprises at least one heat exchanger system at the output.

5. Apparatus according to any of the preceding claims, wherein the heat exchanger at the output of the DMC reactor is followed by at least one DMC condenser and DMC tank for cooling the produced DMC and separating it from the gaseous feed products and gaseous byproducts.

6. Apparatus according to any of the preceding claims, further comprising a MeOH condenser, and MeOH tank downstream of the DMC condenser for cooling the byproduct MeOH and separating it from the gaseous feed products and byproducts, wherein the apparatus optionally comprises a pipe connection between the MeOH tank and a feed pipe of the DMC reactor.

7. Apparatus according to claim 6, wherein the MeOH condenser or tank is connected to a feed pipe of the MeOH reactor by a pipe connection such that the untreated gaseous feed or gaseous byproducts are fed as recycled feed mixture, optionally admixed with fresh feed gas consisting of a mixture mainly comprised of CO, and/or CO₂, and H₂.

8. Apparatus according to claim 7, further comprising an additional bypass line including a CO₂ steam reforming reactor SRR and a pipe connection between the MeOH condenser and the feed pipe of the MeOH reactor.

9. Apparatus according to any of the preceding claims, wherein the MeOH reactor and/or the DMC reactor are shell-tube reactors having one or more tubes which are covered by a shell, and wherein the catalysts are placed inside of the one or more tubes and these tubes are surrounded by a circulating heat transfer media in order to regulate the reactors or zones temperature.

10. Apparatus according to any of the preceding claims, wherein
catalyst C1 is based on CuO/ZnO supported on alumina (Al₂O₃),
catalyst C2 is selected from the group consisting of CeO₂, ZrO₂, ZnO-CeO₂, and any mixture thereof,
catalyst C3 is selected from the group consisting of Pt/γ-Fe₂O₃, Au/γ-Fe₂O₃, Au/CeO₂-MOₓ/Al₂O₃ wherein M is at least one of the following group: La, Ni, Cu, Fe, Cr, and Y; Au-Y/CeO₂, Pt-Y/CeO₂, Au/CeO₂-ZrO₄, Au/Hydrotalcite and Cu-Zn-Al₂O₃, Pt/Hydrotalcite and Cu-Zn-Al₂O₃, Pt/CeO₂.

11. Apparatus according to any of the preceding claims, wherein the preferred composition of the catalysts, in weight percent, are 20 to 35% for C1, 15 to 35% for C2 and 15 to 60% for C3.

12. Process for producing dimethyl carbonate (DMC), comprising the step of synthesizing DMC from a feed mixture at least consisting of MeOH, CO₂, and CO by heterogeneous catalysts in a DMC reactor comprising a mixture of at least two heterogeneous catalyst groups comprising catalysts C2 and C3, wherein catalyst C2 promotes a reaction of MeOH with CO₂ to produce DMC and H₂O as byproduct, and catalyst C3 promotes the transformation of CO and H₂O to H₂ and CO₂.

13. Process according to claim 12, further comprising a step of synthesizing MeOH from a feed mixture at least consisting of CO, CO₂, and H₂, in a MeOH reactor which is either integrally combined within the DMC reactor or is sequentially placed upstream of the DMC reactor to produce MeOH for the DMC synthesis process in the DMC reactor, wherein the step of synthesizing MeOH is a heterogeneous catalytic process catalyzed by a third group of heterogeneous catalysts, namely catalyst C1, which promotes the reaction of CO, H₂ and CO₂ to MeOH.

14. Process according to claim 12 or claim 13, wherein the MeOH reactor is fed with a mixture of H₂ in the range of 3 to 10 wt.%, CO in the range of 40 to 69 wt.% and CO₂ in the range of 15 to 42 wt.%, at pressure conditions from 15 to 60 bars and temperature conditions from 175 to 270 °C for catalyst C1, and the subsequent DMC reactor is fed with the output of the MeOH reactor at pressure conditions from 15 to 60 bars and temperature conditions from 110 to 175°C for catalyst C2 and from 110 to 175 °C for catalyst C3, wherein, when MeOH reactor is integrally combined within the DMC reactor having inside catalysts C1, C2, and C3 mixed together, the MeOH reactor is fed with a mixture of H₂, in the range of 3 to 10 wt.%, CO in the range of 40 to 69 wt.% and CO₂ in the range of 15 to 42 wt.%, while the pressure conditions are from 15 to 60 bars and the temperature conditions are from 130 to 165 °C.

15. Process according to claim 14, wherein the weight hourly space velocity of the feed gas is from 1,200 to 125,000 h⁻¹.
